# EUROPEAN PATENT APPLICATION

(11) **EP 4 473 905 A1**
(43) Date of publication of application: **11.12.2024**
(21) Application number: 23178245.9
(22) Date of filing: 08.06.2023
(51) Int. Cl.: A61B 5/07, A61B 5/145, A61B 5/1473, A61B 5/00

(54) **A DEVICE AND A METHOD FOR ANALYZING A CHARACTERISTIC OF AN ANALYTE IN A TEST LIQUID**

(71) Applicant: Stichting IMEC Nederland, 6708 WH Wageningen (NL)
(72) Inventor: ARMSTRONG, Rachel, 5616 HB Eindhoven (NL); ELIAS, Jinane, 6524 PK Nijmegen (NL); MATHWIG, Klaus, 5611 ND Eindhoven (NL); Zevenbergen, Marcel, 5706 GN Helmond (NL)
(74) Representative: AWA Sweden AB

(57) **Abstract**

According to an aspect of the present inventive concept there is provided a device for analyzing a characteristic of an analyte in a test liquid, the device comprising: a reservoir configured to hold an analysis liquid, the analysis liquid comprising an analysis compound; a microfluidic channel having a first end and a second end, wherein the first end is arranged in fluid communication with the reservoir, wherein the second end is configured to be in fluid communication with the test liquid; wherein the microfluidic channel is configured to allow mixing, by diffusion, of the analysis compound entering the first end with the analyte entering the microfluidic channel from the second end; and a detector arranged in relation to the microfluidic channel, wherein the detector is configured to detect a property dependent on the mixing of the analysis compound and the analyte, said property being representative of the characteristic of the analyte in the test liquid.

## Description

### Technical field

The present description relates to the field of a device for analyzing a characteristic of an analyte in a test liquid and a method for analyzing a characteristic of an analyte in a test liquid.

### Background

Titration is an important method which is used in many different applications such as acid-base titration, redox titration, or Karl Fischer analysis.

Recent development in titration includes automated titration apparatuses, flow injection analysis (FIA), and research in miniaturization of titration instruments, which is driven by the benefits of smaller, portable detection devices (on-site detection or point-of-care detection), and by the ability to handle smaller sample volumes.

However, analytical techniques that quantify analytes in solution often require large instruments and large volumes. Additionally, if the technique requires additional reagents, the mixing of reagents relies on a stirring apparatus or pumps to generate flow. These factors limit the miniaturization potential of many analytical techniques and make them complex. Moreover, state-of-the-art titration techniques only allows for discrete time-point measurements.

Generally, these limitations prevent application of titration methods to beyond a laboratory environment towards at the point of care or remote or on-site sensing such as handheld environmental sensing.

Thus, there is a need for improvements in the art.

### Summary

It is an object of the present description to at least partly overcome one or more limitations of the prior art. It is an object to provide a device for analyzing a characteristic of an analyte in a test liquid. It is a further object to provide a method for analyzing a characteristic of an analyte in a test liquid.

These and other objects are at least partly met by the invention as defined in the independent claims. Preferred embodiments are set out in the dependent claims.

As a first aspect, there is provided a device for analyzing a characteristic of an analyte in a test liquid, the device comprising:
a reservoir configured to hold an analysis liquid, the analysis liquid comprising an analysis compound;
a microfluidic channel having a first end and a second end, wherein the first end is arranged in fluid communication with the reservoir, wherein the second end is configured to be in fluid communication with the test liquid;
   wherein the microfluidic channel is configured to allow mixing, by diffusion, of the analysis compound entering the first end with the analyte entering the microfluidic channel from the second end; and
a detector arranged in relation to the microfluidic channel, wherein the detector is configured to detect a property dependent on the mixing of the analysis compound and the analyte, said property being representative of the characteristic of the analyte in the test liquid.

The device fully relies on a passive system, using diffusion. Diffusion is a process resulting from random motion of molecules by which there is a net flow of matter from a region of high concentration to a region of low concentration. Diffusion is thus driven by concentration gradients. In other words, there is no need for any active mixing, pumping or dispensing for using the device. Thanks to this the device may have a small size. The device may be integrated into miniaturized devices, such as lab-on-chip or implantable or ingestible devices. Further, the device does not require any electricity to power a flow. Thus, the power consumption of the device is very low.

The sample volume needed for the device to run is very low. In other words, small samples may be analyzed

The device may be used for measuring health features within human health.

The characteristic of the analyte may be the concentration of the analyte in the test liquid. Thus, the device may be used for determining a concentration of an analyte in the test liquid.

The device may further be used for kinetic measurements of an analyte of a known concentration. The device may be used for analysis of characteristics of the analyte or test liquid such as diffusivity, viscosity or reactivity.

The device may be used for measuring health features within human health.

The analyte may be a compound found in the human body. Thus, the analyte may be a compound used when measuring health of a human. The analyte may be coupled to for instance pH of an organ of the human body. The analyte is in any case dissolved in the test liquid. The analyte may comprise ions, such as K⁺, Na⁺, NH₄⁺, SO₄²⁻. The analyte may further comprise acids, bases, organic, inorganic, and redox compounds, complexometric compounds, precipitation compounds, biochemical compounds, short-chain fatty acids, enzymes, nitrates, nitrites, heavy metals, or any other compound that can be detected with state-of-the-art titration techniques.

The test liquid may further be water. The water may be dissolved in non-aqueous test liquid. The device may be used for identifying an unknown analyte and any characteristics of the analyte in the water. In other words, the device may be used in a Karl Fisher analysis.

The reservoir is configured to hold an analysis liquid. The analysis liquid comprises an analysis compound. The analysis compound may be a titrant. The analysis compound may be a known compound of a known concentration or pH or any other relevant feature.

The analysis liquid may be a buffer, such as a phosphate buffer. The analysis liquid may further comprise hydrochloric acid, a base, an alcohol, or iodine-compounds, redox compounds, or any compound used as titrant in state-of-the-art titration.

The first end of the microfluidic channel is in fluidic connection with the reservoir. In other words, the analysis liquid of the reservoir may reach the microfluidic channel.

In the same way, the second end of the microfluidic channel is in fluidic connection with the test liquid. The test liquid may be arranged in a further reservoir of the device, or the test liquid may be arranged in the surrounding of the device. In other words, the device may in use be arranged within the test liquid and by that the second end of the microfluidic channel is in fluidic connection with the test liquid.

The microfluidic channel is further configured for allowing mixing of the analysis compound and the analyte. The mixing is made by diffusion. This implies that no active component is necessary for stirring or generating a flow within the device for mixing the analysis compound with the analyte.

Further, the microfluidic channel is configured to allow the analysis compound to enter the microfluidic channel through the first end by diffusion, and the microfluidic channel is configured to allow the analyte to enter the microfluidic channel through the second end by diffusion. This further implies that no active component is necessary for generating a flow of the analysis compound or the analyte within the device.

In other words, the analysis compound is diffusing from the analysis liquid comprised in the reservoir into the microfluidic channel. The analyte is diffusing from the test liquid into the microfluidic channel. The analysis compound and the analyte are diffusing from the first and second end of the microfluidic channel, respectively, and are mixed while diffusing.

It should be further realized that the analysis liquid, not only the analysis compound, may diffuse into the microfluidic channel through the first end. Also, the test liquid, not only the analyte, may diffuse into the microfluidic channel through the second end.

The mixing of the analysis compound and the analyte gives rise to a property dependent on the mixing of the analysis compound and the analyte. In other words, the analysis compound and the analyte enter the microfluidic channel, by diffusion, and diffuse along the microfluidic channel. The analysis compound and the analyte will eventually meet in the microfluidic channel, upon which a mixing will take place. The mixing may give rise to interaction between the analysis compound and the analyte, which may result in the property to be detected. The interaction may be a chemical reaction taking place when the analysis compound and the analyte meets in the microfluidic channel.

Thus, the microfluidic channel may, at a given time point, comprise the analysis compound, the analyte and a reaction product from mixing of the analyte and analysis compound. The forming of the reaction product may imply that the analyte may be consumed by the mixing with the analysis compound within the microfluidic channel such that the analyte may not diffuse through an entire length of the microfluidic channel.

The detector may be configured to detect a property of the analyte, the analysis compound or a reaction product and/or a property of a combination of one or more of the analyte, the analysis compound and the reaction product.

The property is representative of the characteristic of the analyte in the test liquid. The property may have a gradient along the microfluidic channel.

As an example, the characteristic may be a concentration of the analyte in the test liquid. Then, the concentration of the analysis compound is high in the reservoir and low in the test liquid. Therefore, the concentration of the analysis compound continuously decreases in the microfluidic channel from the first end to the second end. At the second end, the analyte diffuses from the test liquid into the microfluidic channel, thus an analyte concentration gradient is established in the microfluidic channel with a concentration that is continuously decreasing in the microfluidic channel from the second end to the first end. Thus, the characteristic, in this example the concentration of the analyte, may be determined by detection of the position of an inflection point of concentration of the analyte, the analysis compound and/or the reaction product along the microfluidic channel. The inflection point is the point at a curve, in this example a curve of the concentration of the analyte, the analysis compound and/or the reaction point along the microfluidic channel, where a second derivative of the curve is zero. The inflection point may be a point where just enough analyte and analysis compound are mixed so as to reach an equilibrium. The characteristic may also or alternatively be determined by detection of a peak position of concentration of the analyte, the analysis compound and/or the reaction product in space, along the microfluidic channel. Further, the characteristic may be determined by detection of a change of the overall amount of analysis compound or reaction product in the microfluidic channel. In other words, depending on the concentration of the analysis compound in the analysis liquid and the analyte in the test liquid, respectively, an inflection point or peak of a compound concentration profile shifts along the microfluidic channel. Such shift may be detected by the detector arranged in relation to the microfluidic channel.

The gradient of the property may be used for calculating different characteristics of the analyte. As an example, if diffusion coefficients of the analyte and the analysis compound are known, the concentration of the analyte may be calculated. If the concentrations of the analyte and the analysis compound are known, the diffusion coefficient may be calculated.

Thus, the mixing of the analysis compound and the analyte may for instance give rise to a mixture having a different color compared to before the mixing. The color change, thus the property, is then measured by the detector. The detector may relate the color measured to the characteristic of the analyte in the test liquid.

Other examples of the property may be changed in pH or temperature.

The device may further comprise a processing unit configured to: receive the property detected by the detector; determine the characteristic of the analyte based on the property. Thus, the processing unit may be able to determine the characteristic of the analyte based on the measured property. The processing unit may have information on the analysis compound, such that the processing unit uses known information of the analysis compound in combination with the property to determine the characteristic.

The processing unit may be arranged in the device. Alternatively, the processing unit may be arranged in a device that is close to the user and which may be configured to communicate through wired or wireless communication with the detector for acquiring the measured property.

This implies that a result of the analysis of the characteristic may be presented to the user in the device, being available to the subject. Hence, the analysis of the characteristic may be quickly presented to the user making analysis of the characteristic available to the subject in real time.

As yet another alternative, the processing unit may be arranged remotely and may be configured to receive the measured property through communication over a telecommunication or data network, such that the processing unit may for instance be arranged "in the cloud".

The first end and the second end may be arranged on opposite sides of the microfluidic channel. In other words, the microfluidic channel may have an elongated path between the first end and the second end. The analysis compound may travel from the first end along the elongated path of the microfluidic channel towards the second end. The analyte may travel from the second end along the elongated path of the microfluidic channel towards the first end. The analysis compound and the analyte are mixed anywhere along the elongated path of the microfluidic channel.

The microfluidic channel may be configured to provide a first diffusion direction for the analysis compound starting from the first end and diffusing towards the second end. The microfluidic channel may further be configured to provide a second diffusion direction for the analyte starting from the second end and diffusing towards the first end. Thus, the analysis compound and the analyte meet during the diffusion in the microfluidic channel to allow mixing. The analysis compound and the analyte may diffuse with different speeds, thus the mixing may be made at any distance from the first end and the second end respectively. In other words, the device may alternatively be used to measure diffusivities. Concentration profiles in the microchannel may also depend on reaction rates or kinetics of chemical reactions. Thus, for sufficiently slow reaction rates of analyte and analysis compound, the device may alternatively be used to measure such reaction rates.

The reservoir may have a volume of 1 µL to 1 L. The reservoir may have a volume of 1 µl to 100 µl, 1 ml to 100 ml, 1 cl to 100 cl, 1 dl to 100 dl or 1 l. The reservoir may have a volume of 10 µl, 100 µl, 10 ml or 100 ml. In other words, the reservoir may have a micro-size or it may be a larger reservoir. Thus, the reservoir may be a small size adapted to be ingested or it may be a larger size for table-top analysis. An advantage of this is that the size of the reservoir may be adapted for a specific use and a specific duration of use.

The cross-section of the microfluidic channel may be 100 nm² to 1 mm². The cross-section of the microfluidic channel may be 1 µm² to 100 µm². The diameter of the microfluidic channel may be 10 nm or 50 µm. Thus, the cross-section of the microfluidic channel may vary depending on the application of use of the device.

The microfluidic channel may have a length of 10 µm to 10 cm. Thus, the length of the microfluidic channel may be 10 µm, 100 µm, 1 mm, 100 mm, or 1 cm. The length of the channel may be adapted for the duration of use of the device.

The volume of the reservoir, the cross-section of the microfluidic channel and the length of the microfluidic channel may all be adapted for the use of the device. Several combinations of the features are possible.

The cross-section of the microfluidic channel may be varied along the length of the channel. In other words, the microfluidic channel may be tapered. Tapering may tune the measured property or gradient of property. Further, tapering may allow an increase in measurement performance. In other words, tapering may give higher resolution and/or precision of the measurement.

The device may further comprise a removable cover arranged to avoid diffusion through the microfluidic channel, wherein the cover is configured such that it opens the microfluidic channel for diffusion to initiate analysis. The removable cover may be arranged at the first end of the microfluidic channel. The removable cover may be arranged at the second end of the microfluidic channel. The removable cover may be arranged inside, along the elongation of the microfluidic channel. The removable cover may comprise a cap, a film, a membrane or a reversible valve. The opening of the cover may be induced by the cover being detached, dissolved or degraded while exposed to a certain environment. For instance, the cover may be dissolved upon a change in pH. This may for instance occur if the device is ingested and while reaching the stomach acid. An advantage of this is that any diffusion into the microfluidic channel is avoided before the device is in the right environment for analysis. Also, the device may be configured to inherently remove the cover at a desired point in time, e.g., when an ingested device is in a desired location. This implies that no active component may be necessary for removing the cover.

The detector may be a chemical sensor, an ion-selective electrode sensor, a chemical field effect transistor, an ion-sensitive field effect transistor, a pH sensor, a pH sensitive dye, a photodiode, a camera, a CMOS sensor, a CCD sensor, a fluorescence sensor, a spectroscope, a colorimetric sensor, or a temperature sensor.

According to a second aspect, there is provided an ingestible device comprising the device according to above. The device may be ingested and used for analysis of any compound present in the stomach of the person ingesting it. The analyte may then be any compound dissolved in the stomach of the person.

The advantages as discussed above are equally valid for the second aspect.

According to a third aspect, there is provided an analysis device for monitoring a biological or chemical process, the analysis device comprising the device according to above, wherein the analysis device is arranged in-line, at-line or on-line of the biological or chemical process.

In an in-line arrangement the analysis device is arranged in the biological or chemical process. Thus, the analysis device may be arranged in a process flow and conduct an analysis in the process flow.

In an at-line arrangement the analysis device is arranged outside of the process flow. For this analysis, a sample may be acquired from the biological or chemical process and presented to the analysis device.

In an on-line arrangement the analysis device is arranged in connection to the biological or chemical process flow, such that the analysis device may conduct automatic sampling from the process flow.

The advantages as discussed above are equally valid for the third aspect. The analysis device may be a versatile device, adapted to be used in many different biological or chemical processes. The size of the analysis device may be adapted for the biological or chemical process.

The device for analyzing a characteristic of an analyte allows for continuous detection of the property. Thus, the device is suitable for use in monitoring of a biological or chemical process as the biological or chemical process may be continuously monitored over a long period of time.

According to a fourth aspect, there is provided a method for analyzing a characteristic of an analyte in a test liquid, the method comprises:
arranging a reservoir holding an analysis liquid in relation to the test liquid, the analysis liquid comprising an analysis compound, wherein the reservoir is connected to a microfluidic channel having a first end and a second end, wherein the first end is arranged in fluid communication with the reservoir, wherein the second end of the microfluidic channel is arranged in fluidic communication with the test liquid such that the analysis compound is diffused, through the first end of a microfluidic channel, into the microfluidic channel; and the analyte is diffused, through the second end of the microfluidic channel, into the microfluidic channel;
mixing the analysis compound with the analyte by the analysis compound and the analyte being diffused into the microfluidic channel; and
detecting, by a detector, a property dependent on the mixing of the analysis compound and the analyte, said property being representative of the characteristic of the analyte in the test liquid.

The advantages discussed for the first aspect are equally valid for the fourth aspect.

The analysis may be performed within seconds. The time of the analysis may be prolonged into hours. The analysis time may be controlled by the length of the microfluidic channel. Further, the analysis time or response time of mixing and detecting may ensure that the method yields the average or mean value of the characteristic of the analyte in the test solution during this time. Thus, short time fluctuations of the characteristics are filtered, and the method may sense a smoothed average magnitude of the characteristic. This is an advantage compared to other methods.

The characteristic may be a concentration of the analyte in the test liquid. The analysis compound may have a known concentration in the analysis liquid for determining the concentration of the analyte in the test liquid. Further, the method may be used for analysis of other characteristics of the analyte or test liquid such as diffusivity, viscosity or reactivity.

The detecting of the property may detect a gradient or profile of the property along a length of the microfluidic channel. Thus, an advantage of having the detector arranged along the microfluidic channel is that diffusivities or transport properties may be measured by the method.

The method may provide continuous detection of the property. Thus, the measurement may continue without any abruption for a short or long time, such that variations of the property may be analyzed over time. This is an advantage over the state-of-the-art titration methods, where measurements are made at discrete time-points requiring extraction of samples at each instance at which a measurement is to be made. Thus, changes over time are not covered by the state-of-the-art methods.

The method allows continuous measurement while the measurement channel is in fluidic communication with the test liquid. Measurements may thus be performed continuously e.g. in-line with a chemical process or during continuous deployment of a measurement device in a test liquid. The test liquid may for instance be a body fluid using an ingestible device which is arranged in fluidic communication with the body fluid. The test liquid may alternatively be a fluid in the environment such that the method may be used e.g. for continuously monitoring water quality by deploying a device in a water source or any body of water.

As the analysis liquid sample volume needed for the device to run is very low, a small sample may be analyzed continuously for a long time.

The analysis may be performed for hours up to weeks, or wherein the analysis may be performed for years. Having a large reservoir and a thin microfluidic channel, the analysis may even be performed with negligible drift for up to centuries. The duration of the analysis may depend on parameters including reservoir volume and geometry of the microfluidic channel. In other words, the method may be used for a long-time measurement of the property. This may be advantageous when analysis of health or environmental or process parameters, since those may need time to change or may fluctuate over time.

### Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.
Fig. 1 is a side view of a device according to an embodiment.
Fig. 2 is a side view of a device according to an embodiment.
Fig. 3 is a side view of an ingestible device according to an embodiment.
Fig. 4 is a side view of an analysis device according to an embodiment.
Fig. 5 is a schematic overview of a method according to an embodiment.

### Detailed description

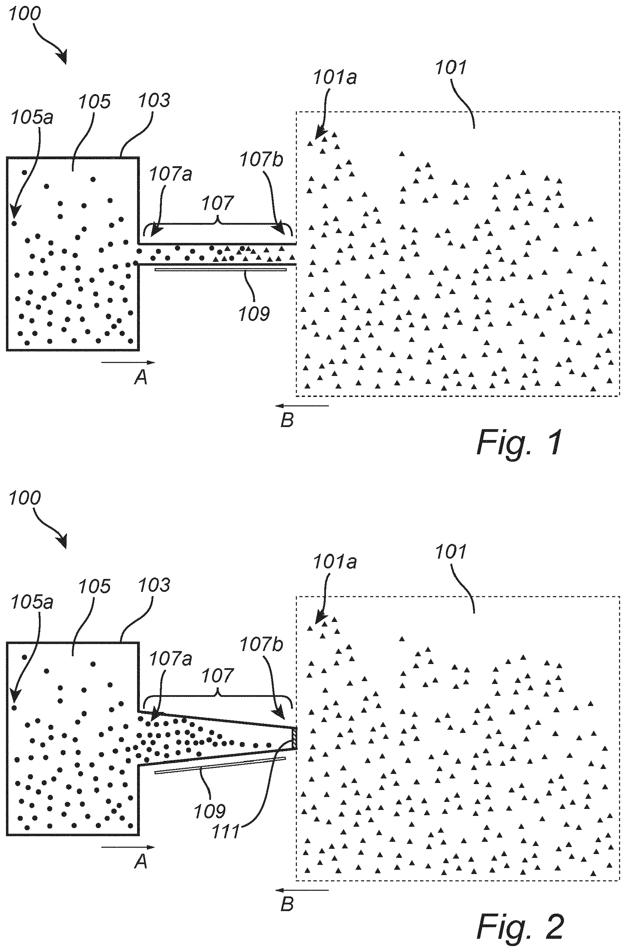

Fig. 1 illustrates a side view of device 100 for analyzing a characteristic of an analyte 101a in a test liquid 101. The analyte 101a may comprise ions, such as K⁺, Na⁺, NH₄⁺, SO₄²⁻. The analyte may further or alternatively comprise acids, bases, organic, inorganic, and redox compounds, complexometric compounds, precipitation compounds, biochemical compounds, short-chain fatty acids, enzymes, nitrates, nitrites, heavy metals, or any other compound that can be detected with titration techniques.

The device 100 comprises a reservoir 103 configured to hold an analysis liquid 105. The analysis liquid 105 comprises an analysis compound 105a. The analysis compound 105a may be a titrant. The analysis compound 105a may be a known compound of a known concentration or pH or any other relevant feature. The analysis liquid 105 may be a buffer, such as a phosphate buffer. The analysis liquid 105 may further comprise hydrochloric acid, a base, an alcohol, iodine-compounds, redox compounds, or any compound used as titrant in state-of-the-art titration.

The reservoir 103 may have a volume of 1 µL to 1 L. The reservoir 103 may have a volume of 1 µl to 100 µl, 1 ml to 100 ml, 1 cl to 100 cl, 1 dl to 100 dl or 1 l. The reservoir 103 may have a volume of 10 µl, 100 µl, 10 ml or 100 ml. In other words, the reservoir 103 may have a micro-size or it may be a larger reservoir. Thus, the reservoir 103 may be a small size adapted to be ingested or it may be a larger size for table-top analysis.

The device further comprises a microfluidic channel 107. The microfluidic channel 107 may have a length of 10 µm to 10 cm, such as a length of 10 µm, 190 µm, 1 mm, 100 mm, or 1 cm. The microfluidic channel 107 may have a cross-section of 100 nm² to 1 mm².

As is shown in Fig. 2, the cross-section of the microfluidic channel 107 may be varied along the length of the channel.

The relation between the length and cross-section of the microfluidic channel and the size of the reservoir 103 will be discussed further in relation to Table 1 below.

The microfluidic channel 107 has a first end 107a and a second end 107b. As is shown in Fig. 1 and Fig. 2 the first end 107a and the second end 107b are arranged on opposite sides of the microfluidic channel 107. However, it should be understood that other geometries are also possible.

The first end 107a is arranged in fluid communication with the reservoir 103. The second end 107b is configured to be in fluid communication with the test liquid 101. The microfluidic channel 107 is configured to allow mixing, by diffusion, of the analysis compound 105a entering the first end 107a with the analyte 101a entering the microfluidic channel 107 from the second end 107b.

The microfluidic channel 107 may be configured to provide a first diffusion direction A for the analysis compound 105a starting from the first end 107a and diffusing towards the second end 107b. The microfluidic channel 107b may further be configured to provide a second diffusion direction B for the analyte 101a starting from the second end 107b and diffusing towards the first end 107a. In other words, the analysis compound 105a enters the microfluidic channel 107 by the first end 107a by diffusion. The analysis compound 105a will diffuse along the microfluidic channel 107 at the direction A. The analyte 101a enters the second end 107b of the microfluidic channel 107 by diffusion. The analyte 101a diffuses along the microfluidic channel 107 at the direction B. At some point of the microfluidic channel 107, the analysis compound 105a and the analyte 101a will meet and start to mix. The mixing will give rise to a property which is representative of the concentration, pH, diffusivity, viscosity or reactivity of the analyte 101a in the test liquid 101. The mixing may give rise to interaction between the analysis compound and the analyte, which may result in forming of a reaction product. The interaction may be a chemical reaction taking place when the analysis compound and the analyte meets in the microfluidic channel.

The device 100 further comprises a detector 109. The detector 109 is arranged in relation to the microfluidic channel 107. As can be seen in Fig. 1 the detector 109 is arranged along the microfluidic channel 107, however it should be understood that the detector may be arranged in other ways in relation to the microfluidic channel 107. The detector 109 may further comprise several detector units 109 arranged in relation to the microfluidic channel 107 such that several different analyses may be made by the different detector units 109. The detector 109 is configured to detect a property dependent on the mixing of the analysis compound 105a and the analyte 101a, said property being representative of the characteristic of the analyte 101a in the test liquid 101. The detector 109 may be a chemical sensor, an ion-selective electrode sensor, a chemical field effect transistor, an ion-sensitive field effect transistor, a pH sensor, a pH sensitive dye, a photodiode, a camera, a CMOS sensor, a CCD sensor, a fluorescence sensor, a spectroscope, a colorimetric sensor, or a temperature sensor. The device may further comprise a processing unit. The processing unit may be configured to receive the detected property. The detected property may be received from the detector 109. The detector 109 may be configured to detect the property based on different signals based on the property and convert the signals to digital values that may be received by the processing unit.

In this regard, the processing unit does not need any special interface for receiving the detected property and the processing unit may be implemented as a general-purpose processing unit, such as a central processing unit (CPU), which may execute the instructions of one or more computer programs in order to process the received detected property. Hence, a computer program product may be provided, which provides computer-readable instructions for causing the processing unit to analyze the detected property. The computer program product may be provided as a signal carrying the computer program product for allowing the computer program product to be loaded into a memory accessible to the processing unit. According to an embodiment, the computer program product may be provided as a non-transient computer program product stored on any tangible media.

The processing unit may alternatively be implemented as firmware arranged e.g. in an embedded system, or as a specifically designed processing unit, such as an Application-Specific Integrated Circuit (ASIC) or a Field-Programmable Gate Array (FPGA), which may be configured to implement functionality for processing the received detected property.

Fig. 2 illustrates a side view of the device 100. There are similarities between Fig. 1 and Fig. 2 which will not be discussed here.

In Fig. 2 the microfluidic channel 107 has a cross-section which is varied along the length of the channel. For instance, the device 100 has a tapered microfluidic channel 107. However, it should be realized that the microfluidic channel 107 may have a cross-section that varies in other ways. The variation may extend of over the entire length of the microfluidic channel 107 or the size of the cross-section may only vary at a certain portion of the length. The variation may further be such that the cross section is larger and smaller several times over the length of the microfluidic channel 107.

Further, Fig. 2 shows that the device 100 comprises a removable cover 111 arranged to avoid diffusion through the microfluidic channel 107. The cover 111 may be configured such that it opens the microfluidic channel 107 for diffusion to initiate analysis. In Fig. 2 the removable cover 111 is arranged at the second end 107b of the microfluidic channel 107. However, it may as well be arranged at the first end 107a of the microfluidic channel or it may be arranged inside at any point of the length of the microfluidic channel 107. The removable cover 111 may comprise a cap, a film, a membrane or a reversible valve. The opening of the cover 111 may be induced by the cover 111 being detached, dissolved or degraded while exposed to a certain environment. For instance, the cover 111 may be dissolved upon a change in pH.

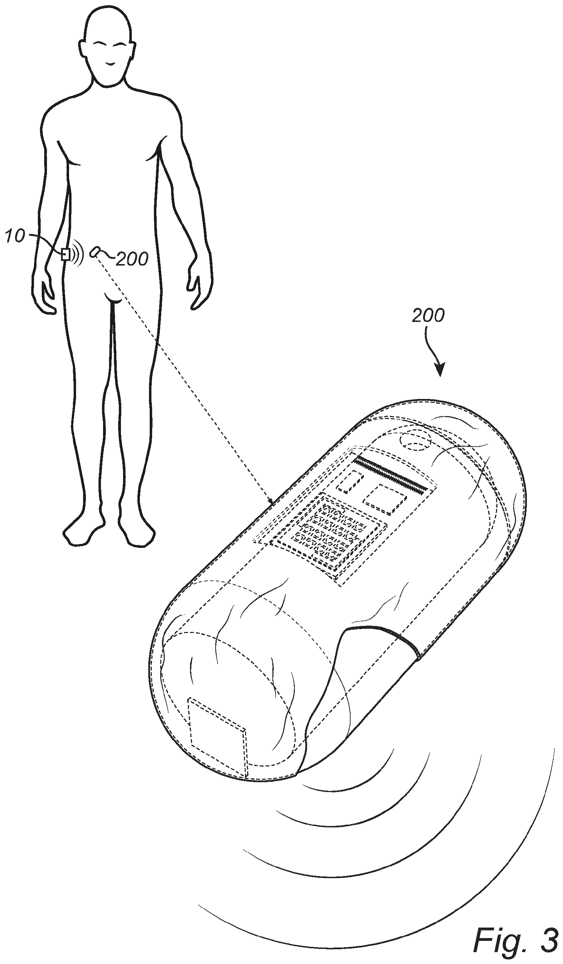

Fig. 3 illustrates an ingestible device 200 comprising the device 100 according to above. The ingestible device 200 may be ingested and used for analysis of any compound or health state of the stomach.

For instance, if the ingestible device 200 has a device 100 comprising a removable cover 111 the removable cover may dissolve upon exposure to the low pH of the stomach.

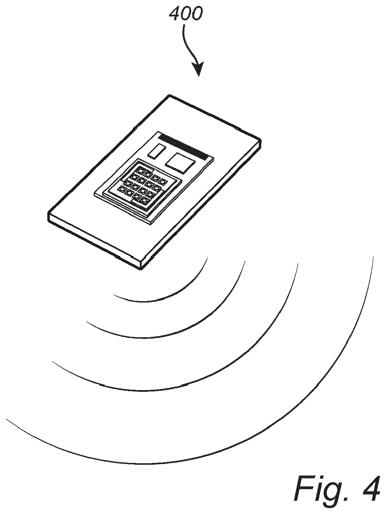

Fig. 4 illustrates an analysis device 400 for monitoring a biological or chemical process. The analysis device 400 comprises the device 100 according to above. The analysis device 400 may be arranged in-line, at-line or on-line of the biological or chemical process.

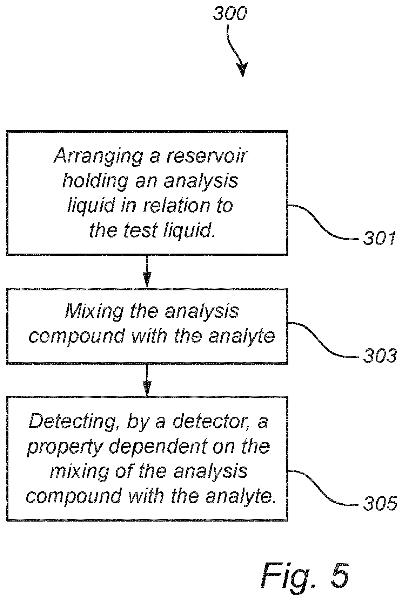

Fig 5 illustrates a schematic view of a method 300 for analyzing a characteristic of an analyte 101a in a test liquid 101. The method 300 comprises arranging 301 a reservoir 103 holding an analysis liquid 105 in relation to the test liquid 101.

The analysis liquid 105 comprises an analysis compound 105a. The analysis compound 105a may have a known concentration in the analysis liquid 105. This may be used when for determining the concentration of the analyte 101a in the test liquid 101, when the characteristic is the concentration. However, the method 300 may be used for analysis of other characteristics of the analyte or test liquid such as diffusivity, viscosity or reactivity. In that case, other characteristics of the analysis compound 105a than the concentration may be known.

The reservoir 103 is connected to a microfluidic channel 107 having a first end 107a and a second end 107b. The first end 107a is arranged in fluid communication with the reservoir 103. The second end 107b of the microfluidic channel 107 is arranged in fluidic communication with the test liquid 101. The analysis compound 105a is diffused, through the first end 107a of the microfluidic channel 107, into the microfluidic channel 107. The analyte 101a is diffused, through the second end 107b of the microfluidic channel 107, into the microfluidic channel 107.

The method further comprises mixing 303 the analysis compound 105a with the analyte 101a by the analysis compound 105a and the analyte 101a being diffused into the microfluidic channel 107.

Further, the method comprises detecting 305 by a detector 109, a property dependent on the mixing 303 of the analysis compound 105a and the analyte 101a. The property is representative of the characteristic of the analyte 101a in the test liquid 101. The detecting 305 of the property may detect a gradient or profile of the property along a length of the microfluidic channel 107.

The method 300 may provide for the analysis to be performed for hours up to weeks, or wherein the analysis may be performed for years. Having a large reservoir 103 and a thin microfluidic channel 107, the analysis may even be performed with negligible drift for up to centuries. The method 300 may provide for continuous detection 305 of the property.

Table 1 shows the relation between the length and the diameter of the microfluidic channel and the size of the reservoir 103 and how it affects the response time (to steady-state concentrations in the microfluidic channel 107) and drift time (defined as change of concentration due to depletion to analysis compound diffusion out of the reservoir 103) of the method 300.

| | | | | 10 µl reservoir | 100 µl reservoir | 10 ml reservoir | 100 ml reservoir |
|---|---|---|---|---|---|---|---|
| Channel length (µm) | Channel diameter (µm) | Diffusivity of analysis compound 10⁻⁹m²/s | Response time (to 99%) (sec) | Time to 10 % drift | Time to 10 % drift | Time to 10 % drift | Time to 10 % drift |
| 150 | 10 | 9.0 | 4.3 | 27 h | 11 days | 3 years | 3 years |
| 150 | 10 | 0.5 | 30 | 200 h | 83 days | 22 years | 22 years |
| 150 | 50 | 9.0 | 6.4 | 66 min | 11 h | 47 days | 47 days |
| 150 | 50 | 0.5 | 45 | 10 h | 100 h | 415 days | 415 days |
| 1000 | 10 | 9.0 | 48 | 7 days | 70 days | 19 years | 19 years |
| 1000 | 10 | 0.5 | 710 | 57 days | 570 days | 156 years | 156 years |
| 1000 | 50 | 9.0 | 49 | 8 h | 33 days | 264 days | 264 days |
| 1000 | 50 | 0.5 | 720 | 54 h | 68 days | 6 years | 6 years |

The method 300 provides an analysis that may be performed within seconds. The start of the analysis may be prolonged into hours. The dimensions of the reservoir 103 and the microfluidic channel 107 may be adapted for the analysis to be made.

Further, the analysis time or response time may yield the average or mean characteristic of the analyte in the test solution during this time. Thus, short time fluctuations of the characteristic are filtered, and the method senses a smoothed average magnitude of the characteristic.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A device (100) for analyzing a characteristic of an analyte (101a) in a test liquid (101), the device (100) comprising:
a reservoir (103) configured to hold an analysis liquid (105), the analysis liquid (105) comprising an analysis compound (105a);
a microfluidic channel (107) having a first end (107a) and a second end (107b), wherein the first end (107a) is arranged in fluid communication with the reservoir (103), wherein the second end (107b) is configured to be in fluid communication with the test liquid (101);
wherein the microfluidic channel (107) is configured to allow mixing, by diffusion, of the analysis compound (105a) entering the first end (107a) with the analyte (101a) entering the microfluidic channel (107) from the second end (107b); and
a detector (109) arranged in relation to the microfluidic channel (107), wherein the detector (109) is configured to detect a property dependent on the mixing of the analysis compound (105a) and the analyte (101a), said property being representative of the characteristic of the analyte (101a) in the test liquid (101).

2. A device (100) according to claim 1, wherein the first end (107a) and the second end (107b) are arranged on opposite sides of the microfluidic channel (107).

3. A device (100) according to claim 1 or 2, wherein the microfluidic channel (107) is configured to provide a first diffusion direction (A) for the analysis compound (105a) starting from the first end (107a) and diffusing towards the second end (107b), and wherein the microfluidic channel (107b) is configured to provide a second diffusion direction (B) for the analyte (101a) starting from the second end (107b) and diffusing towards the first end (107a).

4. A device (100) according to any of the preceding claims, wherein the reservoir (103) has a volume of 1 µL to 1 L.

5. A device (100) according to any of the preceding claims, wherein the cross-section of the microfluidic channel (107) is 100 nm² to 1 mm².

6. A device (100) according to any of the preceding claims, wherein the microfluidic channel (107) has a length of 10 µm to 10 cm.

7. A device (100) according to any of the preceding claims, wherein the cross-section of the microfluidic channel (107) is varied along the length of the channel.

8. A device (100) according to any of the preceding claims, wherein the device (100) further comprises a removable cover (111) arranged to avoid diffusion through the microfluidic channel (107), wherein the cover (111) is configured such that it opens the microfluidic channel (107) for diffusion to initiate analysis.

9. A device (100) according to any of the preceding claims, wherein the detector (109) is a chemical sensor, an ion-selective electrode sensor, a chemical field effect transistor, an ion-sensitive field effect transistor, a pH sensor, a pH sensitive dye, a photodiode, a camera, a CMOS sensor, a CCD sensors, a fluorescence sensors, a spectroscope, a colorimetric sensor, or a temperature sensor.

10. An ingestible device (200) comprising the device according to any one of claims 1 to 9.

11. An analysis device (400) for monitoring a biological or chemical process, the analysis device comprising the device (100) according to any one of claims 1 to 9, wherein the analysis device (400) is arranged in-line, at-line or on-line of the biological or chemical process.

12. A method (300) for analyzing a characteristic of an analyte (101a) in a test liquid (101), the method (300) comprises:
arranging (301) a reservoir (103) holding an analysis liquid (105) in relation to the test liquid (101), the analysis liquid (105) comprising an analysis compound (105a), wherein the reservoir (103) is connected to a microfluidic channel (107) having a first end (107a) and a second end (107b), wherein the first end (107a) is arranged in fluid communication with the reservoir (103), wherein the second end (107b) of the microfluidic channel (107) is arranged in fluidic communication with the test liquid (101) such that the analysis compound (105a) is diffused, through the first end (107a) of a microfluidic channel (107), into the microfluidic channel (107); and the analyte (101a) is diffused, through the second end (107b) of the microfluidic channel (107), into the microfluidic channel (107);
mixing (303) the analysis compound (105a) with the analyte (101a) by the analysis compound (105a) and the analyte (101a) being diffused into the microfluidic channel (107); and
detecting (305), by a detector (109), a property dependent on the mixing (303) of the analysis compound (105a) and the analyte (101a), said property being representative of the characteristic of the analyte (101a) in the test liquid (101).

13. A method (300) according to claim 12, wherein the characteristic is a concentration of the analyte (101a) in the test liquid (101), and wherein the analysis compound (105a) has a known concentration in the analysis liquid (105) for determining the concentration of the analyte (101a) in the test liquid (101).

14. A method (300) according to any one of claims 12 or 13, wherein the detecting (305) of the property detects a gradient or profile of the property along a length of the microfluidic channel (107).

15. A method (300) according to any one of claims 12 to 14, wherein the method (300) provides continuous detection (305) of the property.
